# EUROPEAN PATENT SPECIFICATION

(11) **EP 2 379 066 B1**
(45) Date of publication and mention of the grant of the patent: **26.03.2014**
(21) Application number: 08807092.5
(22) Date of filing: 16.09.2008
(51) Int. Cl.: A61K 31/485, A61P 27/02

(54) **USE OF OPIOID ANTAGONISTS FOR THE PREPARATION OF A MEDICAMENT IN THE TREATMENT OF RETINAL DEGENERATIVE DISEASES**
VERWENDUNG VON OPIOID ANTGONISTEN ZUR HERSTELLUNG EINES ARZNEIMITTELS ZUR BEHANDLUNG VON DEGENERATIVEN NETZHAUTERKRANKUNGEN
UTILISATION D'ANTAGONISTES DES OPIACÉS POUR LA PRÉPARATION D'UN MÉDICAMENT DANS LE TRAITEMENT DE MALADIES DÉGÉNÉRATIVES DE LA RÉTINE

(43) Date of publication of application: 26.10.2011
(73) Proprietor: Imuneks Farma Ilaç Sanayi Ve Ticaret A.S., 34349 Istanbul (TR)
(72) Inventor: PISAK, Ibrahim Mustafa Iskender, Gayrettepe ISTANBUL 34349 (TR); SELAMOGLU, Mehmet Levent, Gayrettepe ISTANBUL 34349 (TR); PAK, Nevhiz, Gayrettepe ISTANBUL 34349 (TR); BINGOL, Semra, Gayrettepe ISTANBUL 34349 (TR)
(74) Representative: Kodron, Felix
(86) International application number: PCT/IB2008/002412
(87) International publication number: WO 2010/032073

(56) References cited:
- NI YING-QIN ET AL: "Neuroprotective effects of naloxone against light-induced photoreceptor degeneration through inhibiting retinal microglial activation" IOVS, vol. 49, no. 6, June 2008 (2008-06), pages 2589-2598, XP002550374 ISSN: 0146-0404
- LAM TIM T ET AL: "The effects of naloxone on retinal ischemia in rats" JOURNAL OF OCULAR PHARMACOLOGY, MARY ANN LIEBERT, INC. NEW YORK, NY, US, vol. 10, no. 2, 1 January 1994 (1994-01-01), pages 481-492, XP009124102 ISSN: 8756-3320
- ZAGON ET AL: "Naltrexone accelerates healing without compromise of adhesion complexes in normal and diabetic corneal epithelium" BRAIN RESEARCH BULLETIN, ELSEVIER SCIENCE LTD, OXFORD, GB, vol. 72, no. 1, 13 February 2007 (2007-02-13), pages 18-24, XP005885659 ISSN: 0361-9230
- FISCHER A J ET AL: "OPIATE AND N-METHYL-D-ASPARTATE RECEPTORS IN FORM-DEPRIVATION MYOPIA" VISUAL NEUROSCIENCE, CAMBRIDGE UNIVERSITY PRESS, GB, vol. 15, no. 6, 1 November 1998 (1998-11-01), pages 1089-1096, XP009024602 ISSN: 0952-5238
- GUPTA KALPNA ET AL: "Morphine exaggerates retinopathy in transgenic sickle mice." BLOOD, vol. 106, no. 11, Part 1, November 2005 (2005-11), pages 64A-65A, XP002550375 & 47TH ANNUAL MEETING OF THE AMERICAN-SOCIETY-OF-HEMATOLOGY; ATLANTA, GA, USA; DECEMBER 10 -13, 2005 ISSN: 0006-4971
- COOK H L ET AL: "Age-related macular degeneration: diagnosis and management" BRITISH MEDICAL BULLETIN, vol. 85, March 2008 (2008-03), pages 127-149, XP002550376 ISSN: 0007-1420
- DELYFER MARIE-NOELLE ET AL: "Inherited retinal degenerations: therapeutic prospects" BIOLOGY OF THE CELL (PARIS), vol. 96, no. 4, May 2004 (2004-05), pages 261-269, XP002550377 ISSN: 0248-4900

## Description

### Background of the invention

The present invention lies in the field of pharmacy and is dealing with the preparation of a new medicament for the therapy of retinal degenerative diseases.

The retina which is the light sensitive portion of the eye, is a complex tissue that contains specialized photoreceptor cells called rods and cones. The photoreceptors connect to a network of nerve cells for the local processing of visual information. This information is sent to the brain for decoding into a visual image. The rods are mostly located away from the center of the eye in the retinal periphery. The highest concentration of cones is found at the center of the retina, the macula, which is necessary for visual acuity.

Under the retina is the choroid, a collection of blood vessels embedded within a fibrous tissue, and the pigmented epithelium (PE), which overlays the choroid layer. The retinal pigment epithelium (RPE) cells provide support, protection, and nutrition to the light sensitive retina, and cooperate with other retinal cells to maintain normal visual function. The dysfunction and/or loss of these RPE cells play a critical role in the development of the vision loss.

RPE cells are key to the survival of the photoreceptor cells which are particularly susceptible to injury since they are often the first cells to degenerate or suffer damage as a result of a traumatizing event or condition. Hereditary defects in specific genes, retinal detachment, circulatory disorders, overexposure to light, toxic effects to drugs and nutritional deficiencies are among the wide array of causes that can result in the death of photoreceptors cells.

The retina is susceptible to a variety of diseases, including age related macular degeneration, retinitis pigmentosa, diabetic retinopathy and other inherited retinal degenerations, uveitis, retinal detachment, and eye cancers.

Retinitis pigmentosa (RP) designates a group of inherited diseases that affect the retina and are characterized by a gradual destruction of the rods and cones, resulting in a progressive loss of vision and, possibly, blindness. Usually, the rod cells are the first to degenerate, causing night blindness and 'tunnel vision'. Loss of central vision late in the course of the disease may occur in some cases. RP is often diagnosed during childhood or early adulthood. The rate of progression varies. To date, there is no known way to halt the degeneration of the retina or to cure the disease.

The leading cause of visual loss among elderly persons is macular degeneration, often called AMD or ARMD (age related macular degeneration), signs of which appear after the age of 50. As documented in the western world it is a leading cause of permanent visual loss with a prevalence of 8.5% in persons under 54 years of age and of 37% in persons over 75 years of age. Life expectancy steadily increasing all over the world, vision loss associated with AMD is a growing problem.

AMD occurs with degeneration of the macula, which is the part of the retina responsible for the sharp, central vision needed to read or to drive. It is diagnosed as either dry (non-neovascular) or wet (neovascular). Neovascular refers to growth of new blood vessels in the macula, where they are not supposed to be. The dry form is more common than the wet one, with about 85-90 percent of the patients diagnosed. The wet form of the disease usually leads to more serious vision loss.

Dry AMD as an early stage of the disease and may result from the aging and thinning of macular tissues, depositing of pigment in the macula or a combination of the two processes. Dry macular degeneration is diagnosed when yellowish spots known as drusen begin to accumulate from deposits or debris from deteriorating tissue primarily in the area of the macula. In about 10 percent of cases, dry AMD progresses to a more advanced and damaging wet form when new blood vessels grow beneath the retina and leak blood and fluid. This leakage causes permanent damage to light-sensitive retinal cells, which die off and create blind spots in central vision.

Besides affecting older populations, AMD appears to occur in whites and females in particular. The disease also can result as a side effect of some drugs. It also appears to run in families. Commonly named risk factors include: smoking, high blood pressure, lighter eye color,obesity and inactivity.

Drusen are tiny yellow or white accumulations of extracellular material that build up in Bruch's membrane of the eye. The presence of a few small drusen is normal with advancing age and most people over 40 have some hard drusen. Whether drusen promote AMD or are symptomatic of an underlying process that causes AMD is not clearly known. However an increase in the size and/or number of drusen raises a person's risk of developing macular degeneration.(Ref 1)

Drusen is thought to be associated with the metabolic processes occurring throughout the body. The source of the proteins and lipids in drusen is also not quite clear with potential contributions by both the retinal pigment epithelium (RPE) and the choroid.

Several trace elements are present in drusen **(*Ref 2*)**, the most concentrated being zinc. (***Ref 3*)**

The protein composition of drusen includes apolipoproteins and members of the complement system. Zinc has been suggested to play a role in drusen formation by precipitating and inhibiting the elements of the complement cascade. ***(Ref 3)*** The presence of molecules that regulate inflammation has led some investigators to conclude that drusen are product of the human immune system.**(*Ref 4***)

The treatment of macular degeneration has been disappointing to date. At best, most available treatments help to avoid further vision loss rather than to improve vision. Currently, there is no treatment that can reverse its course. Several clinical trials are underway. (***Ref* 5**)

At present, there are two main interventions.

Age Related Eye Disease Study (AREDS) conducted by the National Eye Institute showed that treatment with antioxidants and zinc reduces the probability of developing advanced macular degeneration as well as moderate vision loss. Many researchers and eye care practitioners believe that certain nutrients - zinc, lutein, zeaxanthin and vitamins A, C and E - help lower the risk for AMD or slow down the progression. Second phase of AREDS study began in late 2005 to evaluate whether similar protective effects against the disease might be associated with other nutrients such as omega-3 fatty acids as well as lutein and zeaxanthin found in leafy vegetables.

The second intervention is the treatment of neovascularization. FDA approved treatments for wet macular degeneration include laser (thermal or photodynamic therapy-PDT) and drugs such as Macugen® (pegaptanib) or Lucentis® (ranibizumab).

There might be many factors related to the etiology of macular degeneration. The macula has very high metabolic needs compared to other areas of the body. It requires proper nutritional elements, oxygen, and the elimination of waste products in order to function properly. The most common causes may be due to the age related changes in the metabolic processes of the human body.

The complement system is a major protein network in blood that has been traditionally conceived as part of the immune system, a proinflammatory cascade engaged in nonspecific antimicrobial defence. However, it became clear recently that this system also plays an essential role in specific, adaptive immune responses, as well as in many basic physiological processes. Complement proteins are widely involved in the immune evasion tactics of infectious microbes and cancer cells, and derangement of complement function contributes to numerous autoimmune, allergic and acute catastrophic diseases.

Being a very ornate system that links the innate and acquired immunity systems together, it consists of a series of about 25 proteins that work to "complement" the work of antibodies in destroying bacteria. The biological functions of the complement system are mediated by an enzymatic reaction cascade involving three pathways (classical, alternative and lectin) which are initiated by distinct mechanisms.

Complement deficiency is a condition of absent or suboptimal functioning of the complement system proteins. These deficiencies may be congenital or acquired and can be divided into two categories.
i) Disorders of the proteins which act to activate the complement system can lead to an underactive response, causing greater susceptibility to diseases.
ii) Disorders of the proteins which act to inhibit the complement system can lead to an overactive response. The complement system has the potential to be extremely damaging to host tissues meaning its activation must be tightly regulated.

It is thought that the complement system might play a role in many diseases with an immune component, such as Barraquer-Simons Syndrome, asthma, lupus erythematosus, glomerulonephritis, various forms of arthritis, autoimmune heart disease, multiple sclerosis, inflammatory bowel disease, and ischemia-reperfusion injuries. It is also becoming increasingly implicated in diseases of the central nervous system such as Alzheimer's disease, and other neurodegenerative conditions.

Recently, the gene of complement factor H, one of the complement system negative regulation proteins, has been determined to be strongly associated with a person's risk for developing macular degeneration. A tyrosine-histidine change at amino acid 402 in complement factor H (CFH) on chromosome 1 results in the formation of a CFH gene variant. People whose genetic makeup includes this variant of the CFH gene are more likely to develop macular degeneration. US data indicated the odds of developing the disease are increased by about 2.5 to 5.5 times.

CFH gene is involved in regulating the inflammatory pathways - alternate complement pathway. The protein encoded by the CFH gene variant increases AMD risk by failing to bind to receptors on the cells of the retina and the choroid which prevents it from inhibiting the inflammatory pathway. Unchecked, the pathway would cause inflammation in the retina and the surrounding blood vessels.

In conclusion, being part of the alternative pathway, CHF is specifically involved with the inhibition of the complement cascade in combination with other proteins. Hence a mutation in CFH will undoubtely increase inflammation and its consequences. **(*Ref 6)***

Finally, for diseases other than AMD, several patents illustrate claims for the inhibition of the complement system. US Pat No 6,248,365 proposes the use of CI inactivators or of factors I or H for the prophylaxis and the therapy of chronic inflammatory intestinal disorders, skin disorders and purpura. US Pat No 6,355,245 proposes the use of monoclonal antibodies to treat glomerulonephritis. US Pat No 6,538,028 proposes the administration of platelet activity modulator for inhibiting the complement activation.

Naltrexone was originally approved in the 80's to help addicts come off opioids and alcohol by totally blocking the brain receptors that trigger the effects of these narcotics. To this end naltrexone is usually available in dosages of 50mg tablets to assist persons wishing to kick their habits.

However recently it has become more apparent that administrated at much lower doses (3- 5mg/day), naltrexone has a significant effect in normalizing the immune system. For example, the November 13, 2003 issue of the New England Journal of Medicine stated as follows: "Opioid-Induced Immune Modulation. The preclinical evidence indicates overwhelmingly that opioids alter the development, differentiation, and function of immune cells, and that both innate and adaptive systems are affected. Bone marrow progenitor cells, macrophages, natural killer cells, immature thymocytes and T cells and B cells are all involved. The relatively recent identification of opioid-related receptors on immune cells makes it even more likely that opioids have direct effects on the immune system*."*

In fact, Bernard Bihari had discovered in 1985 the effects of low dose of naltrexone (LDN) on the body's immune system. He found that LDN was able to enhance a patient's response to HIV infection. In the mid-90's, Dr. Bihari observed that patients in his practice with cancer could benefit, in some cases dramatically, from low dose naltrexone treatment. US Pat No 6,384,044 shows a method of treating cancer of the prostate.

At low doses, naltrexone exerts an opiate blocking action substantially exclusively for Mu opiate receptors over Delta opiate receptors. That is the prescribed low dose regimen such that naltrexone does exert a substantial blocking action for Mu opiate receptors but does not exert such an action against other opiate receptors.

The brief blockade of opioid receptors sites between 2 a.m. and 4 a.m. caused by taking LDN at bedtime is believed to produce a prolonged up-regulation of the body's immune system by inducing an increase in endogeneous opioid peptides production. Normal volunteers who have followed LDN regimen have been found to have higher levels of beta-endorphins in their blood in the following days.

Animal research **(Ref 7**) by Ian Zagon and his colleagues has shown a marked increase in Met-enkephalin levels, another endogeneous opioid peptide. Their cancer research in laboratory studies has demonstrated inhibition of different human tumors by using low dose naltrexone methodology. It has been suggested that the increased endorphin and enkephalin levels induced by LDN work directly on the tumors' opioid receptors and cause cancer cell death by apoptosis. The same opioid peptides are also believed to increase natural killer cells and other healthy immune defenses against cancer.

In general, with diseases that are partially or largely triggered by a deficiency of endorphins (including cancer and autoimmune diseases), or are accelerated by a deficiency of endorphins (such as AIDS), restoration of the body's normal production of endorphins seems to be the major therapeutic action of LDN methodology.

But not only naltrexone is of special medical interest but also naloxone has been tested in a therapy of a special retinal disease.

Ni Ying-Qin et al. (**Ref. 8**) showed by experiments on rats eyes that naloxone can be used to reduce light-induced photoreceptor degeneration through inhibiting retinal microglial activation. Treatment with 1 mg/d naloxone provided significant protection of photoreceptors against photic injury in the superior and inferior quadrants of the retina.

But no improvement effects in the quality of vision have been presented but neuroprotective effects only.

### Summary of the invention

It is thought that for some reason in macular degeneration (AMD) patients, abnormal regulation of the complement cascade occurs at a local level within Bruch's membrane and adjacent retinal pigment epithelial cells (RPE) resulting in uncontrolled compliment activation and consequent drusen formation. Similarly, peripheral photoreceptor cell loss in retinitis pigmentosa (RP) is believed to occur due to systemic activation of the innate immune system.

By normalizing derangements in the human complement system that occurs in the retinal degenerative disease etiology, this invention proposes a therapeutic strategy based on the use of opioid antagonists for the preparation of a medicament for the selective blocking of the body's opioid receptors sites.

### Detailed description of the invention

Surprisingly it has been found that macular degeneration even when in advanced stage can respond to a therapeutical approach involving selective blocking of the patient's opioid receptors sites.

The selectively blocking of the patient's opioid receptors sites is reached by the use of opioid antagonists or their pharmaceutically acceptable salts and derivatives consisting of naltrexone, naloxone and nalmefene at a dosage of from 1 to 10 mg, preferably 4.5 mg per day for internal uses, and from 0,5% to 10% w/w, preferably 1% w/w for external uses, in one or divided doses, preferably in the evening hours for the preparation of a medicament for treating human eye retinal degenerative diseases consisting of age-related macular degeneration and retinitis pigmentosa.

The preferred choice as the active substance in the manufacture of the medicament is naltrexone or its pharmaceutically acceptable salts and derivatives.

Another favourable active substance in the manufacture of the medicament is naloxone or its pharmaceutically acceptable salts and derivatives.

It is also possible that as the active substance nalmefene or its pharmaceutically acceptable salts and derivatives is used in the manufacture of the medicament.

The use of the medicament with opioid antagonists such as naltrexone, naloxone or nalmefene as active substance is aimed to macular degeneration (AMD) and Retinitis pigmentosa (RP).

The pharmaceutical compositions of the present invention may be manufactured in a manner that is itself known, e.g., by means of conventional mixing, dissolving, granulating, dragee-making, levigating, emulsifying, encapsulating, entrapping or tabletting processes.

Pharmaceutical compositions for use in accordance with the present invention thus may be formulated in conventional manner using one or more physiologically acceptable carriers comprising excipients and auxiliaries which facilitate processing of the active compounds into preparations which can be used pharmaceutically. Proper formulation is dependent upon the route of administration chosen. Any of the well-known techniques, carriers, and excipients may be used as suitable and as understood in the art.

The therapeutic agent may be given orally.

For oral administration, the therapeutic agent may be given in the form of tablets, coated tablets, chewable tablets, dispersable tablets, rapid dissolve tablets, sublingual tablets, effervescent tablets, effervescent granules, dragees, capsules, sachets, cachets, caplets, lozenges, liposomes, powders, granules, powders for suspensions, powders for solutions, solutions, emulsions, liquids, syrups and the like.

In preparing the compositions in oral dosage form, any of the usual pharmaceutical media may be employed. Thus for solid oral preparations , such as for example, powders, tablets and capsules, suitable carriers and additives include starches, sugars, diluents, granulating agents, lubricants, binders, disintegrating agents and the like. Because of their case in administration, tablets and capsules represent the most advantageous oral dosage unit form, in which case, solid pharmaceutical carriers are obviously employed. If desired, tablets may be sugar coated or enteric coated by standard techniques. For liquid oral preparations, such as, for example, suspensions, elixirs and solutions, suitable carriers and additives include water, glycols, oils, alcohols, flavoring agents, preservatives, coloring agents and the like.

The therapeutic agent may also be given parenterally.

For parenteral administration, the therapeutic agent may be presented in aqueous solution, for example in the form of a water-soluble salt, or aqueous injection suspensions that contain appropriate liquid carriers, suspending agents and the like. Formulations for injection may be presented in unit dosage forms, e.g. in ampoules or in multi-dose containers. For parenterals, the carrier will usually comprise sterile water, though other ingredients, for example, for purposes such as aiding solubility or for preservation, may be included. The therapeutic agent, optionally together with excipients, can also be given in the form of a lyophilisate and can be made into a solution prior to parenteral adminis-tration by the addition of suitable solvents.

Parenteral delivery may include intramuscular, subcutaneous, intravenous injections.

The therapeutic agent may also be applied topically in the form of creams, ointments, gels, eye drops, lotions, suppositories, enemas and the like, depending on the internal or external use of the pharmaceutical composition.

Daily administration dose to the patient varies from about 0,5 mg to about 10 mg, preferably 0,5 mg to 5 mg/day, in one or divided doses and most preferably at about 4.5 mg per day. These amounts are based on naltrexone specifically, but equivalent amounts can be readily determined for other opiate antagonists which are essentially pure in their antagonistic action at very low dosage levels. It is also preferred that the administration takes place during the evening hours, particularly at the bed time in accordance with regular LDN treatment. The therapy may be followed intermittently or on a regular basis depending on the patient's conditions.

For internal usage, the pharmaceutical composition is administered orally or parenterally at dose levels of 0.5 mg to 10 mg per day, preferably between 0.5 mg - 5 mg in one dose at evening hours.

For external use, the therapeutic composition is prepared in a concentration of 0.5 % to 10 %; preferably 1% w/w.

It is also possible that the medicament contents beside the opioid antagonist such as naltrexone, naloxone or nalmefene other pharmacologically effective drugs or substances.

Low dose naltrexone methodology is targetting modulatory effects of endogeneous opioid peptides on the body's immune system. It seems that the therapy primarily opposes the complement cascade elimination process by MAC activation. Such an immune system attack causes the accumulation of extracellular debris in the macula consequently leading to drusen formation. However, other immune system related biochemical mechanisms may as well come to play to explain the observed therapeutic action.

If other opiate receptors sites antagonists become available and receive governmental approval, such drugs, at least in principle, qualify for application in the present method.

Furthermore, one may administer the drug in a targeted drug delivery system, for example, in a liposome coated with a tissue-specific antibody. The liposomes will be targeted to and taken up selectively by the organ.

### Examples

The following examples provide a detailed illustration of the method of present invention. However they are not intented to limit or restrict the scope of the invention in any way, and should not be construed as providing dosage forms, regimens or methods of administration which must be utilized exclusively to practice the invention.

### Example- 1

An over 80 year old man has had age related macular degeneration for the last 11 years of his life, experiencing progressive burring in the center of his vision. The degeneration led to loss of central vision preventing him from many daily activities. It made him very difficult to lead an independent life. He started on low dose naltrexone regimen , 4.5 mg per day at bedtime. 50-60 days after the initiation of the therapy, he began to experience an improvement in the quality of his vision; he started to differentiate objects around him and movements on the television screen.

### Example-2

An over 85 year old man has had age related macular degeneration for the last 10 years of his life. He experienced a loss of central vision preventing him to differentiate the colors of the surrounding objects and letters in the newspapers. He started on using naltrexone, 4.5 mg per day at bedtime. Approximately 50 days after the initiation of naltrexone usage, the quality of his vision started to improve. He began to differentiate the colors of the objects shown to him; he even started to differentiate the big printed letters in the newspapers.

### References

1- Risk factors associated with age-related macular degeneration - A case-control study in the age-related eye disease study: Report number 3 . Ophthalmology, 107 (12), 2224 - 2232
2- Van der Schaft TL, de Bruijn WC, Mooy CM, Ketelaars DA, de Jong PT (1992). " Element analysis of the early stages of age-related macular degeneration". Arch. Ophthalmol., 110 (3): 389-94.
3- Lengyel I, Flinn JM, Peto T, et al (2007). "High concentration of zinc in sub-retinal pigment epithelial deposits". Exp. Eye Res., 84 (4): 772-80.
4- Hageman GS, Luthert PJ, Victor Chong NH, Johnson LV, Anderson DH, Mullins RF (2001). "An integrated hypothesis that considers drusen as biomarkers of immune-mediated processes at the RPE-Bruch's membrane interface in aging and age-related macular degeneration". Prog Retin Eye Res 20 (6): 705-732.
5- The eye digest", (2007) Eye &.Ear Infirmary, University of Illinois
6- Patel N, Adewoyin T, Chong NV, (2008) "Age-related macular degeneration: a perspective on genetic studies", Eye, 22( 6):768-776.
7- Zagon IS, Rahn KA, McLaughlin PJ , (2007) "Opioids and migration, chemotaxis, invasion, and adhesion of human cancer cells". Neuropeptides, 41 (6) : 441- 452.
8- Ni Ying-Qin et al.:"Neuroprotective effects of naloxone against light-induced photoreceptor degeneration through inhibiting retinal microglial activation" IOVS, vol. 49, no. 6, June 2008 (2008-06)

## Claims

1. The use of opioid antagonists or their pharmaceutically acceptable salts and derivatives consisting of naltrexone, naloxone or nalmefene at a dosage of from 1 to 10 mg, preferably 4.5 mg per day for internal uses, and in a concentration of from 0,5% to 10% w/w, preferably 1% w/w for external uses, in one or divided doses, preferably in the evening hours for the preparation of a medicament for treating human eye retinal degenerative diseases consisting of age-related macular degeneration or retinitis pigmentosa by selectively blocking the patient's opioid receptors sites.

2. The use of opioid antagonists according to claim 1, wherein naltrexone is the active substance of the medicament.

3. The use of opioid antagonists according to claim 1, wherein naloxone is the active substance of the medicament.

4. The use of opioid antagonists according to claim 1, wherein nalmefene is the active substance of the medicament.

5. The use of opioid antagonists according to the claim 1 wherein the therapy is aimed to macular degeneration (AMD) and retinitis pigmentosa (RP).

6. The use of opioid antagonists according to the claim 1 wherein the opioid antagonist such as naltrexone, naloxone or nalmefene is administred to the patient orally.

7. The use of opioid antagonists according to claim 1 wherein the opioid antagonist such as naltrexone, naloxone or nalmefene is administred to the patient in an oral dosage form comprising a tablet, coated tablets, chewable tablets, dispersable tablets, rapid dissolve tablets, sublingual tablets, effervescent tablets, effervescent granules, dragees, capsules, sachets, cachets, caplets, lozenges, liposomes, powders, granules, powders for suspensions, powders for solutions, solutions, emulsions, liquids and syrups.

8. The use of opioid antagonists according to claim 1 wherein the opioid antagonist such as naltrexone, naloxone or nalmefene is administred to the patient parenterally.

9. The use of opioid antagonists according to claim 1 wherein the opioid antagonist such as naltrexone, naloxone or nalmefene is administred to the patient subcutaneous, intramuscular or intraveneous.

10. The use of opioid antagonists according to claim 1 wherein the opioid antagonist such as naltrexone, naloxone or nalmefene is administred to the patient topically in the form of creams, ointments, gels, eye drops, lotions, suppositories and enemas.

11. The use of opioid antagonists according to the preceding claims wherein the medicament is administred daily to the patient, at a dosage of 1-10 mg of the opioid antagonist for internal uses, most preferably at about 4.5 mg per day, and in a concentration of 0,5%-10% for external uses, preferably 1% w/w, in one or divided doses, preferably in the evening hours, for the best receptor selective blocking activity.

12. The use of opioid antagonists according to the preceding claims wherein the medicament contains beside the opioid antagonist such as naltrexone, naloxone or nalmefene other pharmacologically effective drugs or substances.

## Patentansprüche

1. Die Verwendung von Opioidantagonisten oder ihren pharmazeutisch akzeptablen Salzen und Derivaten, bestehend aus Naltrexon, Naloxon oder Nalmefen in einer Dosierung von 1 bis 10 mg, vorzugsweise 4,5 mg pro Tag für die innerliche Anwendung, und in einer Konzentration von 0,5 bis 10 Gewichtsprozent, vorzugsweise 1 Gewichtsprozent für die äußerliche Anwendung, in einer Dosis bzw. mehreren Gaben, vorzugsweise in den Abendstunden für die Zubereitung eines Medikaments zur Behandlung von degenerativen Erkrankungen der menschlichen Netzhaut, also altersabhängige Makuladegeneration oder Retinitis pigmentosa, durch selektives Blockieren der Bereiche der Opioidrezeptoren des Patienten.

2. Die Verwendung von Opioidantagonisten gemäß Anspruch 1, wobei Naltrexon die aktive Substanz des Medikaments ist.

3. Die Verwendung von Opioidantagonisten gemäß Anspruch 1, wobei Naloxon die aktive Substanz des Medikaments ist.

4. Die Verwendung von Opioidantagonisten gemäß Anspruch 1, wobei Nalmefen die aktive Substanz des Medikaments ist.

5. Die Verwendung von Opioidantagonisten gemäß Anspruch 1, wobei die Therapie auf die Makuladegeneration (AMD) und die Retinitis pigmentosa (RP) abzielt.

6. Die Verwendung von Opioidantagonisten gemäß Anspruch 1, wobei der Opioidantagonist, z. B. Naltrexon, Naloxon oder Nalmefen dem Patienten oral verabreicht wird.

7. Die Verwendung von Opioidantagonisten gemäß Anspruch 1,wobei der Opioidantagonist, z. B. Naltrexon, Naloxon oder Nalmefen, dem Patienten in einer oralen Darreichungsform verabreicht wird, also als Tablette, Lacktablette, Kautablette, dispergierbare Tablette, schnell aufzulösende Tablette, sublinguale Tablette, Brausetablette, Brausegranulat, Dragee, Kapsel, Sachet, Cachet, Filmtablette, Pastille, Liposom, Pulver, Granulat, Pulver für Suspensionen, Pulver für Lösungen, Lösung, Emulsion, Flüssigkeit und Sirup.

8. Die Verwendung von Opioidantagonisten gemäß Anspruch 1, wobei der Opioidantagonist, z. B. Naltrexon, Naloxon oder Nalmefen, dem Patienten parenteral verabreicht wird.

9. Die Verwendung von Opioidantagonisten gemäß Anspruch 1, wobei der Opioidantagonist, z. B. Naltrexon, Naloxon oder Nalmefen dem Patienten subkutan, intramuskulär oder intravenös verabreicht wird.

10. Die Verwendung von Opioidantagonisten gemäß Anspruch 1, wobei der Opioidantagonist, z. B. Naltrexon, Naloxon oder Nalmefen, dem Patienten topisch in Form von Cremes, Salben, Gels, Augentropfen, Lotionen, Zäpfchen und Klistieren, verabreicht wird.

11. Die Verwendung von Opioidantagonisten gemäß den vorangehenden Ansprüchen, wobei dem Patienten das Medikament täglich verabreicht wird, und zwar in einer Dosis von 1-10 mg des Opioidantagonisten für innerliche Anwendungen, vorzugsweise etwa 4,5 mg pro Tag und in einer Konzentration von 0,5 % bis 10 % für die äußerliche Anwendung, vorzugsweise 1 Gewichtsprozent, in einer Dosis bzw. mehreren Gaben, vorzugsweise in den Abendstunden, für die beste selektive rezeptorblockende Aktivität.

12. Die Verwendung von Opioidantagonisten gemäß den vorangehenden Ansprüchen, wobei das Medikament neben dem Opioidantagonisten, z. B. Naltrexon, Naloxon oder Nalmefen, weitere pharmakologisch wirksame Arzneimittel oder Substanzen enthält.

## Revendications

1. L'utilisation d'antagonistes opioïdes ou de leurs sels et dérivés pharmaceutiquement acceptables comprenant la naltrexone, la naloxone ou le nalméfène avec un dosage de 1 à 10 mg, de préférence 4,5 mg par jour en usage interne, et avec une concentration de 0,5 % à 10 % p/p, de préférence 1 % p/p en usage externe, en doses uniques ou en doses divisées, de préférence le soir, pour la préparation d'un médicament pour le traitement de maladies dégénératives de la rétine de l'oeil humain dont la dégénérescence maculaire liée à l'âge ou la rétinopathie pigmentaire, agissant par le blocage sélectif des sites des récepteurs opioïdes du patient.

2. L'utilisation d'antagonistes opioïdes selon la revendication 1, **caractérisée en ce que** la naltrexone est la substance active du médicament.

3. L'utilisation d'antagonistes opioïdes selon la revendication 1, **caractérisée en ce que** la naloxone est la substance active du médicament.

4. L'utilisation d'antagonistes opioïdes selon la revendication 1, **caractérisée en ce que** le nalméfène est la substance active du médicament.

5. L'utilisation d'antagonistes opioïdes selon la revendication 1, **caractérisée en ce que** le traitement est destiné à la dégénérescence maculaire (DMLA) et à la rétinopathie pigmentaire (RP).

6. L'utilisation d'antagonistes opioïdes selon la revendication 1, **caractérisée en ce que** l'antagoniste opioïde tel que la naltrexone, la naloxone ou le nalméfène, est administré au patient par voie orale.

7. L'utilisation d'antagonistes opioïdes selon la revendication 1, **caractérisée en ce que** l'antagoniste opioïde tel que la naltrexone, la naloxone ou le nalméfène, est administré au patient avec une forme galénique orale incluant un comprimé, comprimés enrobés, comprimés à croquer, comprimés dispersibles, comprimés à dissolution rapide, comprimés sublinguaux, comprimés effervescents, granulés effervescents, dragées, capsules, sachets, cachets, caplets, pastilles, liposomes, poudres, granulés, poudres pour suspensions, poudres pour solutions, solutions, émulsions, liquides et sirops.

8. L'utilisation d'antagonistes opioïdes selon la revendication 1, **caractérisée en ce que** l'antagoniste opioïde tel que la naltrexone, la naloxone ou le nalméfène, est administré au patient par voie parentérale.

9. L'utilisation d'antagonistes opioïdes selon la revendication 1, **caractérisée en ce que** l'antagoniste opioïde tel que la naltrexone, la naloxone ou le nalméfène, est administré au patient par voie sous-cutanée, intramusculaire ou intraveineuse.

10. L'utilisation d'antagonistes opioïdes selon la revendication 1, **caractérisée en ce que** l'antagoniste opioïde tel que la naltrexone, la naloxone ou le nalméfène, est administré au patient localement sous forme de crèmes, pommades, gels, collyres, lotions, suppositoires et lavements.

11. L'utilisation d'antagonistes opioïdes selon les revendications précédentes, **caractérisée en ce que** le médicament est administré quotidiennement au patient, avec un dosage de 1-10 mg de l'antagoniste opioïde en usage interne, de préférence d'environ 4,5 mg par jour, et avec une concentration de 0,5 %-10 % en usage externe, de préférence 1 % p/p, en doses uniques ou en doses divisées, de préférence le soir, pour la meilleure activité de blocage sélectif du récepteur.

12. L'utilisation d'antagonistes opioïdes selon les revendications précédentes, **caractérisée en ce que** le médicament contient, outre l'antagoniste opioïde tel que la naltrexone, la naloxone ou le nalméfène, d'autres drogues ou substances pharmacologiquement efficaces.
